(19) Europäisches Patentamt
European Patent Office
Office européen des brevets

(11) Numéro de publication : **0 489 071 B1**

(12) # FASCICULE DE BREVET EUROPEEN

(45) Date de publication du fascicule du brevet : **10.11.93 Bulletin 93/45**

(51) Int. Cl.$^5$ : **B01J 31/24**

(21) Numéro de dépôt : **90912758.1**

(22) Date de dépôt : **10.08.90**

(86) Numéro de dépôt international : **PCT/FR90/00607**

(87) Numéro de publication internationale : **WO 91/02588 07.03.91 Gazette 91/06**

(54) **PERFECTIONNEMENT A LA PREPARATION DE CATALYSEURS CHIRAUX A BASE DE COMPLEXES DU RUTHENIUM ET DU PHOSPHORE.**

(30) Priorité : **23.08.89 FR 8911159**

(43) Date de publication de la demande : **10.06.92 Bulletin 92/24**

(45) Mention de la délivrance du brevet : **10.11.93 Bulletin 93/45**

(84) Etats contractants désignés : **AT BE CH DE DK ES FR GB IT LI LU NL SE**

(56) Documents cités :
EP-A- 0 000 315
EP-A- 0 218 970
EP-A- 0 218 970
EP-A- 0 271 311
EP-A- 0 275 354
EP-A- 0 307 168
US-A- 4 397 787

(73) Titulaire : **SOCIETE NATIONALE ELF AQUITAINE**
**Tour Elf, 2, Place de la Coupole, La Défense 6**
**F-92400 Courbevoie (FR)**

(72) Inventeur : **JUGE, Sylvain**
**15, sentier de Châteaufort**
**F-91400 Orsay (FR)**
Inventeur : **GENET, Jean-Pierre**
**70, rue Estienne-D'Orves**
**F-91370 Verrières-le-Buisson (FR)**
Inventeur : **MALLARI, Sergio**
**8, avenue des Chênes**
**F-91400 Orsay (FR)**

(74) Mandataire : **Clisci, Serge et al**
**S.A. FEDIT-LORIOT CONSEILS EN PROPRIETE INDUSTRIELLE 38, avenue Hoche**
**F-75008 Paris (FR)**

**EP 0 489 071 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention se rapporte à la préparation de catalyseurs optiquement actifs, formés par des complexes du ruthénium, dont le ligand est un dérivé organique du phosphore. Elle concerne en particulier des catalyseurs d' hydrogénation asymétrique et d'isomérisation de double liaison ; permettant l'obtention de produits organiques à chiralité voulue. Le procédé suivant l'invention rend possible l'obtention de produits de pureté optique améliorée dans des cas où celle-ci n'est pas suffisante lorsqu'on applique les catalyseurs obtenus selon la technique antérieure. Les catalyseurs, formés par le procédé suivant l'invention, conviennent très avantageusement à la préparation d'acides aminés, et particulièrement de la L-thréonine, par hydrogénation des cétones correspondantes, et en général de composés portant un carbonyle et une fonction amine ou amide.

On connaît, à l'heure actuelle, différents complexes phosphinés du Ru, utilisés comme catalyseurs d'hydrogénations asymétriques. Tel est, par exemple, le bis-(méthyl-2 allyl)bis(triphénylphosphine)-Ru décrit par J. POWELL et B.L. SHAW dans Journ. Chem. Soc. n°A, 1968. pages 159-161. Dans la littérature sur ce sujet, on désigne les ligands organophosphorés, utiles, par des abréviations telles que DIOP, DIPAMP, CHIRAPHOS, BINAP etc. qui sont généralement des phosphines, comme par exemple

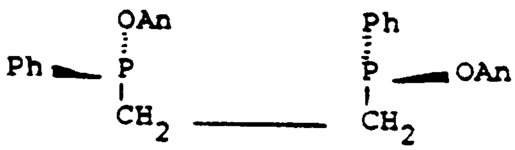 R,R-DIPAMP

soit bis(o-anisyl phénylphosphino)-1,2 éthane, où OAn représente le groupe o-anisyle. Ces ligands sont en général combinés avec un halogénure ou une combinaison organique de Ru, notamment groupe carboxylique ou insaturé, en particulier allylique.

Ainsi R. NOYORI et coll. - J. Amer. Chem. Soc. 1987, 109, pages 5856-5858 - ont-ils décrit l'hydrogénation asymétrique de plusieurs esters carboxyliques portant une fonction cétone en position β , transformant cette dernière en un hydroxyle suivant le schéma

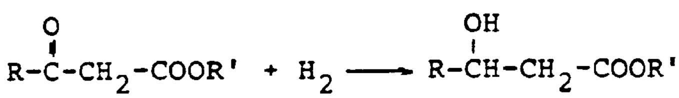

le catalyseur étant $RuX_2$[(R ou S) BINAP] où BINAP désigne la diphosphine

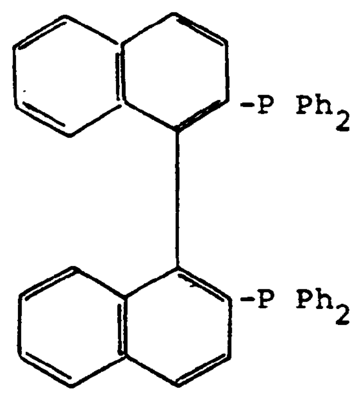

X étant Cl, Br ou I.

Suivant la configuration R ou S de ce ligand, les auteurs ont obtenu le β hydroxy-ester principalement de l'une ou l'autre de ces configurations, la pureté énantiomérique étant toujours très élevée.

De même, M. KITAMURA et coll., dans J. Am. Chem. Soc. 1988, 110, pages 629-631, ont rapporté des hydrogénations asymétriques, semblables, avec le même catalyseur, en opérant avec des corps dans lesquels le $-CH_2-COOR'$ de la formule donnée plus haut est remplacé par $-CH_2-N(R')_2$, $CH_2-OH$, $CH_2-CH_2OH$, $CH_2-COSR'$, $-CH_2-COR'$, $-CH_3CO$, $-C_6H_4COOH$, $CH_3$; $-CO-CH_3$, $-CH_2-COR'$ ou

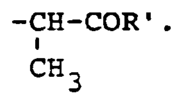

Dans ces cas encore, les rendements de l'hydrogénation sont en général fort élevés et la pureté optique (excès énantiomérique %, e.e) très haute.

Il s'avère cependant que, si l'on cherche à réaliser les mêmes réactions avec des catalyseurs, dans lesquels le groupe BINAP est remplacé par un groupe plus économique, par exemple un de ceux dont il est question plus haut, les résultats deviennent aléatoires et moins bons. Les travaux, dont dérive la présente invention, font penser que cet inconvénient serait dû à l'altération subie par les produits au cours de la préparation du catalyseur, qui comprend le chauffage d'une combinaison du ruthénium avec une phosphine. On peut lire, en effet, dans l'article de TETSUO OHTA et coll. - Inorg. Chem. 1988, 27, page 567, ou dans celui de Takao IKARIYA et coll. J. Chem. Soc., Chem. Commun. 1985, page 922 , que, pour préparer des complexes de Ru avec du BINAP, on chauffe un composé du ruthénium avec de la phosphine BINAP, dans du toluène, en présence de triéthylamine, à reflux, donc au voisinage de 100°C, durant 12 heures. Si la tétraphényl dinaphtyl diphosphine, qui constitue le BINAP, et son produit de réaction résistent bien à ce traitement, il n'en serait plus de même pour les différentes autres phosphines, plus fragiles. L'intérêt de la présente invention réside en ce que des catalyseurs très actifs, à base de complexes du Ru avec des ligands phosphiniques, plus économiques que ceux des articles précités, peuvent être préparés industriellement dans des conditions plus douces, par un nouveau procédé perfectionné.

Selon le procédé suivant l'invention, un complexe du ruthénium avec , à la fois, un diène et un composé allylique est chauffé avec une phosphine au sein d'un liquide organique, inerte vis-à-vis des substances en présence, de façon à substituer la phosphine au diène, dans le complexe du ruthénium ; ce procédé est caractérisé en ce que le produit obtenu est soumis à l'action d'un acide dans un solvant organique.

Divers acides, minéraux ou organiques, conviennent à la réalisation du procédé, tels que - par exemple - hydracides, acides sulfurique, sulfoniques, perchlorique, phosphoriques, fluorosulfonique, acétique, propioniques, benzoïque et autres. Particulièrement pratique est l'utilisation des hydracides, notamment HCl, HBr, HI ou $HBF_4$, surtout HBr.

Selon un mode opératoire préféré, le complexe allylique de Ru-phosphine étant dissous dans un solvant organique approprié, on agite la solution avec l'acide préalablement dilué dans un liquide organique, le même que ledit solvant ou différent.

Il est bon d'employer la solution de l'acide à une concentration de 0,2 à 3N,et de préférence de 0,5 à 1,5 N, la quantité d'acide étant d'au moins 2 équivalents par atome de Ru présent, de préférence 2 à 4 équivalents.

La solution de complexe, ainsi agitée avec l'acide, est, de préférence, maintenue à une température de 0° à 40°C, la température ambiante, notamment de 15° à 25°C,étant tout à fait favorable. Ce traitement est prolongé jusqu'à l'élimination du composé allylique qui est remplacé, dans le complexe, par l'anion de l'acide utilisé. Suivant la nature des substances en présence, selon celle de l'acide, la concentration de celui-ci, et la température, le traitement acide, suivant l'invention, dure en général environ 1/4 d'heure à 6 heures, et le plus souvent 1 à 3 heures.

La réaction, produite par le traitement acide suivant l'invention, peut se schématiser comme suit :

(1)..

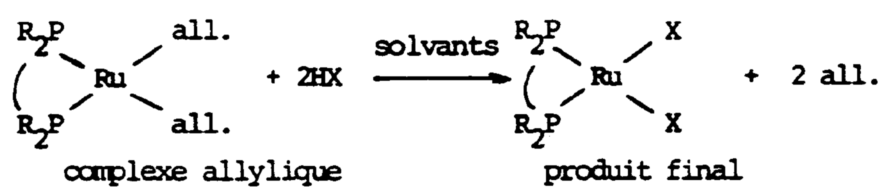


(all. désignant le composé allylique)

La préparation d'un complexe du ruthénium avec un diène, l'allylation de ce complexe et sa réaction avec une phosphine, pour donner la matière de départ de l'opération (1) ci-dessus, sont connues dans l'art, il n'y a donc pas lieu de les décrire ici. On rappellera seulement que le complexe Ru-diène peut s'obtenir par l'action d'un sel de Ru, par exemple $RuCl_3$, sur un diène en $C_4$ à $C_{16}$, de préférence cyclique, comme cyclohexadiène, cycloheptadiène, cyclooctadiène, para-menthadiène, $\alpha$ -phellandiène, norbornadiène, etc.

L'allylation, également connue en soi, peut être effectuée à l'aide de différents composés oraano-métalliaues allyliques

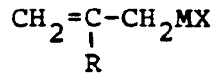

ou R-CH=CH-$CH_2$-MX ,
où M est un métal, généralement Mg, X un halogène et R un atome de H ou un groupe hydrocarboné, le plus souvent en $C_1$ à $C_6$ ; comme l'allyle est éliminé dans l'opération finale, suivant l'invention, il y a intérêt à employer le composé le moins coûteux ; aussi, dans la pratique se sert-on en général du groupe allyle (R=H) ou méthallyle (R=$CH_3$).

Le remplacement du diène par un ligand phosphine, dans le complexe allylé, s'effectue, dans ses grandes lignes, comme dans la technique antérieure, mais le chauffage a lieu à une température moins élevée et pendant un temps plus court. Ainsi, une caractéristique avantageuse du procédé de l'invention réside en ce que le complexe allylique phosphiné, soumis au traitement acide, a été préparé par un chauffage, ne dépassant pas 80°C, du complexe diène-Ru-allyle avec une phosphine et la température est de préférence comprise entre 60°C et 80°C. D'autre part, ce chauffage n'est pas prolongé au-delà de 6 heures, le temps préféré étant de 3 à 5 heures.

On voit que les conditions opératoires sont bien moins sévères que dans la technique connue (article de TETSUO OHTA cité plus haut) où l'on chauffe durant 12 heures, bien au-dessus de 80°C.

A titre d'exemple non limitatif, on décrit ci-après une suite d'opérations effectuées en vue de l'obtention d'un catalyseur complexe suivant l'invention.

I. Formation d'un complexe cyclooctadiène-$RuCl_2$

La réaction de $RuCl_3$ avec le diène peut s'écrire :

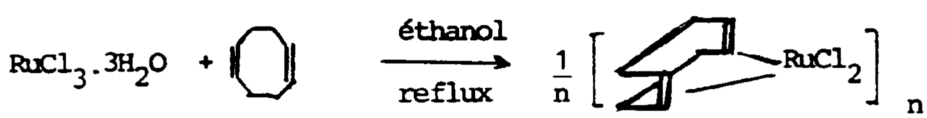


...I

n est le degré de polymérisation du complexe.
Dans 80 g d'éthanol on dissout 2,45 g de $RuCl_3.3H_2O$ et on ajoute 8 g de cyclooctadiène. Le milieu est porté à reflux pendant 3 jours ; la solution obtenue est filtrée et laisse 2,6 g d'un solide marron qui est le complexe cyclooctadiène-$RuCl_2$ à 100%.

II. Allylation du complexe cyclooctadiène-$RuCl_2$

Le complexe obtenu en I est mis à réagir avec du magné- sien de méthylallyle

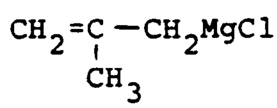

dans de l'éther pour donner :

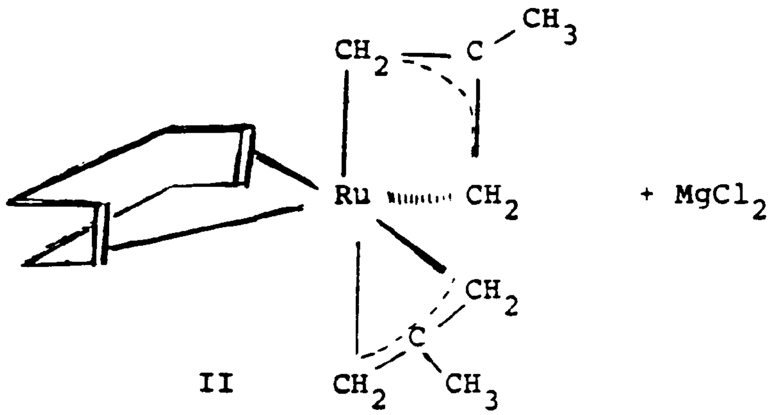

II

Pour cela, les 2,6 g (9,3 mmol) de complexe, obtenu plus haut, sont mis dans 150 ml d'éther et mélangés avec 120 ml d'une suspension dans l'éther de 48 mmoles de chlorure de méthyl allyl magnésium, soit une suspension 0,4 M en magnésien.

Il y a 5,16 moles de magnésien par mole de complexe cyclooctadiène-Ru.

Le mélange est agité pendant 6 heures à 20°C, puis filtré sur "Celite". Dans le filtrat, refroidi à 0°C, on hydrolyse l'excès de magnésien de méthyl allyle à l'aide de 100 ml d'eau glacée.

La phase aqueuse est décantée et traitée trois fois par 100 ml d'éther chaque fois, en vue de l'extraction du complexe allylé II formé. Les phases organiques sont réunies, séchées sur du $CaCl_2$, filtrées et concentrées. Le résidu est repris par 9 ml de benzène et la solution obtenue est passée sur une colonne d'alumine ; après élution avec 150 ml de benzène et évaporation de ce solvant,on obtient 2,7 g d'un solide blanc à 97% de complexe allylé II.

III - Phosphination

Le diène est remplacé,dans le complexe II, par une phosphine :

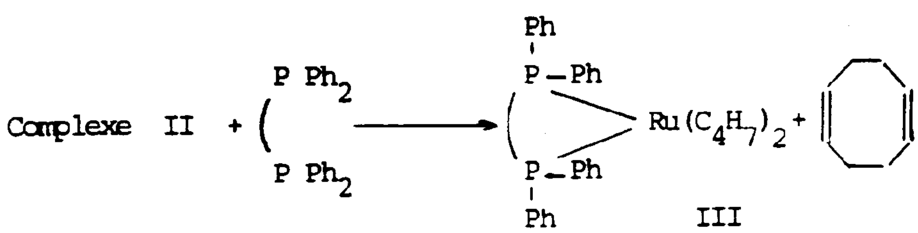


III

La réaction a lieu dans un appareil de Schlenck où l'on introduit 200mg de cyclohexadiène-Ru-bis(methallyle) obtenu lors de l'étape précédente, et 3ml d'hexane ou toluène/hexane, dégazé par un courant d'argon. Après quoi, on ajoute la diphosphine voulue à raison de 1 mole par atome de Ru présent. La solution est maintenue à une température de 60° à 80°C, pendant 3 à 5 heures. Le complexe formé précipite alors dans l'hexane. Après refroidissement, le solvant surnageant est prélevé à l'aide d'une seringue. Le reste est éliminé à la pompe à palettes, et l'on recueille le produit solide formé.

Ce mode opératoire a été appliqué aux ligands connus sous les dénominations suivantes : Diop, Binap, Univerphos, Norphos, Prophos, Deguphos, Dipamp, Bnpe, Dimpc, Bppm.

IV - Traitement à l'acide

Cette opération supplémentaire, suivant l'invention, a pour but le remplacement des groupes allyliques sur le ruthénium par des anions, comme indiqué plus haut à propos de la réaction (1).

Dans l'exemple spécifique, non limitatif, où les anions de l'acide sont des Cl, on a dissous le produit solide, résultant de l'opération III ci-dessus, dans 50ml de dichlorométhane sec, dégazé. A cette solution on a ajouté 37ml d'une solution de HCl N dans du méthanol, soit 37 mmoles d'HCl pour les 9 mmoles de complexe III présent.

Le mélange est agité pendant 2 heures à la température ambiante, après quoi on fait évaporer le solvant,

ce qui laisse un solide rouge brun, directement utilisable comme catalyseur.

Dans un autre exemple, où le contre-ion est l'acétate ($CH_3CO_2^{\ominus}$), on a dissous 50mg de produit solide, résultant de l'opération III ci-dessus, dans 2ml de toluène anhydre, dégazé. A cette solution on a ajouté 2 équivalents, par atome de Ru, d'acide acétique en solution dans le toluène (2M). Le mélange est agité pendant 2 heures à la température ambiante, après quoi on évapore le solvant sous vide. On obtient un solide directement utilisable comme catalyseur de réduction de C=C. De cette manière on a préparé par exemple du Diop Ru $(OAc)_2$, Dipamp $Ru(OAc)_2$, Univerphos Ru $(OAc)_2$.

Avec différentes phosphines, une série de plusieurs catalyseurs a été préparée par le procédé suivant l'invention . D'autre part, avec les mêmes phosphines ont été synthétisés des catalyseurs correspondants suivant la technique antérieure,notamment par l'action d'une diphosphine sur un complexe diène-$RuCl_2$ comme indiqué par TETSUO OHTA dans l'article signalé plus haut.

Afin d'estimer la valeur des catalyseurs, on les a essayés dans l'hydrogénation asymétrique de l'acéto-3 acétamido-2 butyrate de méthyle de façon à produire l'hydroxy-3 acétamido-2 butyrate de méthyle facile à transformer en thréonine. En choisissant le catalyseur de chiralité appropriée, on obtient la thréonine L particulièrement recherchée pour son utilité comme adjuvant de l'alimentation d'animaux. Les essais d'hydrogénation, suivis de deux autres traitements, comprenaient les réactions suivantes :

(a) 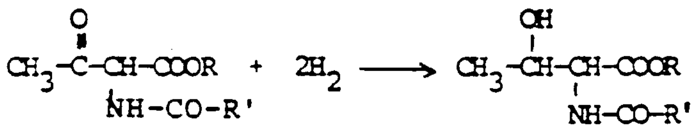

(b) 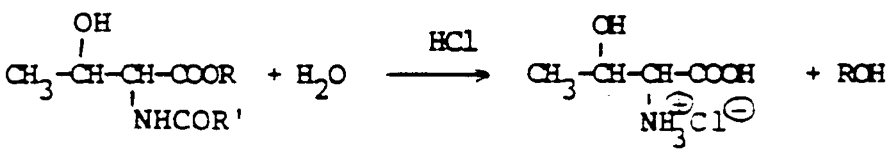


(c) 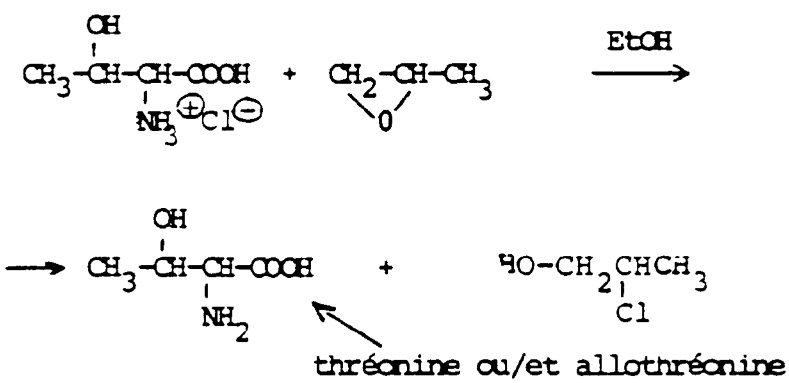


Des essais effectués, il résulte que R et R', semblables ou différents,peuvent être des radicaux très variés, surtout des alkyles en $C_1$ à $C_{12}$ ou/et des aryles en $C_6$ à $C_{10}$; comme ils sont éliminés par les réactions (b) et (c),il est préférable qu'ils soient les plus économiques possible, donc avant tout des $CH_3$ ou des $C_2H_5$.

Pour effectuer l'hydrogénation (a) à l'abri de l'air, on introduisait, dans un autoclave, 1mmole de butyrate susmentionné, 1 ml d'un solvant dégazé, généralement THF, $CH_3OH$ ou $C_2H_5OH$, ainsi qu'une quantité de catalyseur telle qu'il y ait 1 atome de Ru pour 100 moles de butyrate. L'autoclave était rempli d'hydrogène sous 40 bars, et vidé au moins 4 fois, après quoi une pression d'hydrogène de 90 bars était maintenue pendant 48 heures. On chassait alors le solvant sous vide et on déterminait le taux de conversion du -CO en -CHOH par la RMN $^1H$ ou $^{13}C$.

L'hydrolyse (b) consistait à mettre le produit hydrogéné en suspension dans 3 ml d'une solution aqueuse de HCl 3N et à porter celle-ci à reflux pendant 3 heures. Le liquide était ensuite évaporé sous vide et l'on recueillait le solide restant.

Ce dernier était soumis à l'alcoolyse (c). Pour cela il était repris par 4 ml d'éthanol et 2 ml d'oxyde de propylène et chauffé à reflux durant 15 minutes. Après l'évaporation du solvant, on reprenait le produit avec 2 ml

d'acétone; une poudre blanche était séparée par filtration de la solution acétonique obtenue.

Dans le produit recueilli on déterminait la teneur, % en poids, en thréonine et l'excès énantiomérique % en l'isomère optique L,intéressant ; le reste était généralement constitué par de l'allothréonine.

Les résultats de ces essais sont réunis au Tableau donné plus loin, où sont indiqués les catalyseurs employés et:

R-Cat. - rendement en catalyseur préparé selon chacune des 2 méthodes appliquées ;
R-H - rendement de l'hydrogénation sur le catalyseur obtenu par chacune de ces méthodes ;
Thr % - % pondéral de thréonine dans le produit hydrogéné ,
e.e. % - excès énantiomérique % en L-thréonine.

Le terme "INVENTION" désigne le catalyseur obtenu par le nouveau procédé, objet de la présente description, tandis que "ART ANTERIEUR" se rapporte au catalyseur préparé à la manière classique, notamment par le procédé selon TETSUO OHTA et coll. cité plus haut.

Les formules et les noms commerciaux suivants correspondent aux catalyseurs figurant au Tableau des résultats de la page qui suit.

EXEMPLE 1

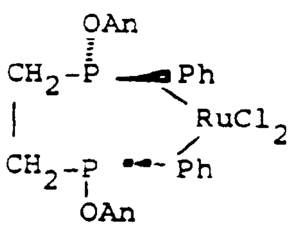

"DIPAMP" $RuCl_2$

EXEMPLE 2

"DIPAMP" Ru $Br_2$

EXEMPLE 3

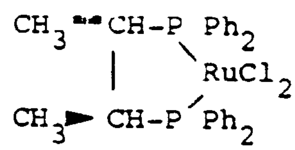

"CHIRAPHOS" $RuCl_2$

EXEMPLE 4

"CHIRAPHOS" Ru $Br_2$

EXEMPLE 5

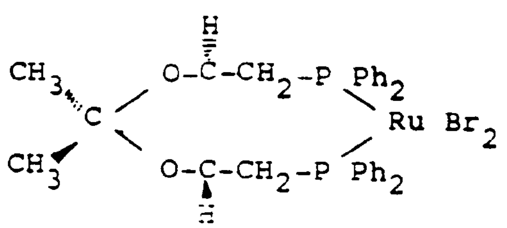

"DIOP" Ru $Br_2$

| Ex. N° | CATALYSEUR | INVENTION | | | | ART ANTERIEUR | | | |
|---|---|---|---|---|---|---|---|---|---|
| | | R-Cat. | R-H | Thr % | e.e. % | R-Cat. | R-H | Thr % | e.e. % |
| 1 | bis(o-anisyl phényl phosphino)-1,2 éthane-Ru $Cl_2$ "DIPAMP" | 76% | 80% | 88 | 30 | 27% | 80% | 80 | 0 |
| 2 | " " " Ru $Br_2$ "DIPAMP" | 81% | 90% | 80 | 32 | 29% | 80% | 79 | 0 |
| 3 | bis(diphényl phosphino butane)-Ru $Cl_2$ "CHIRAPHOS" | 78% | 90% | 71 | 56 | 15% | - | - | - |
| 4 | " " " Ru $Br_2$ "CHIRAPHOS" | 82% | 91% | 95 | 74 | 17% | - | - | - |
| 5 | O,O-isopropylidène-2,3 dihydroxy-2,3 bis (diphényl phosphino)-1,4 butane-Ru $Br_2$ "DIOP" | 76% | 89% | 70 | 53 | 12% | - | - | - |

On voit que le procédé de l'invention permet l' obtention de catalyseurs au P et Ru à base de différentes phosphines avec bons rendements ; ces catalyseurs donnent, à l'hydrogénation, des résultats très convenables, les produits obtenus pouvant titrer notamment 70 à 95% de thréonine, et il est possible d'atteindre des teneurs élevées en L-thréonine (e.e. = 30 à 74%) Par contre, appliquée aux mêmes phosphines que le procédé de l'invention, la méthode connue (TETSUO OHTA cité plus haut) donne le catalyseur avec de mauvais rendements l'impureté des produits ainsi obtenus fait que - dans certains cas (exemples 2 à 5) - il n'est même pas possible d'effectuer l'hydrogénation correctement, ce qui explique le manque de données dans le Tableau de la page précédente. Dans les exemples 1 et 2, où l'on a tout-de-même pu utiliser le catalyseur préparé à la manière classique, on n'obtient aucun enrichissement en l'isomère optique L de la thréonine (e.e. = O), contrairement à ce qui est réalisé suivant l'invention.

**Revendications**

1. Procédé de préparation d'un catalyseur chiral, constitué par un complexe organique du ruthénium et du phosphore, qui comprend le chauffage d'un complexe de ruthénium avec une phosphine, au sein d'un liquide organique, inerte vis-à-vis des substances en présence,le complexe chauffé étant à base d'un diène et d'un composé allylique, caractérisé en ce que le produit obtenu à la suite de ce chauffage est soumis à l'action d'un acide dans un solvant organique.

2. Procédé suivant la revendication 1, caractérisé en ce que l'acide est minéral ou organique, en particulier hydracide, acide sulfurique, sulfonique, perchlorique, phosphorique, fluorosulfonique, acétique, propionique ou benzoïque.

3. Procédé suivant la revendication 2, caractérisé en ce que l'acide préféré est HCl, HBr, HI ou $HBF_4$.

4. Procédé suivant une des revendications 1 à 3, caractérisé en ce que la solution de l'acide dans son solvant est 0,2 à 3N, et de préférence 0,5 à 1,5N, en l'acide.

5. Procédé suivant une des revendications 1 à 4, caractérisé en ce que la quantité d'acide employé est d'au moins 2 équivalents par atome de Ru présent, et de préférence 2 à 4 équivalents.

6. Procédé suivant une des revendications précédentes, caractérisé en ce que le traitement à l'acide a lieu à une température de 0° à 40°C et, en particulier, entre 15° et 25°C pendant 1/4 d'heure à 6 heures.

7. Procédé suivant une des revendications précédentes, caractérisé en ce que le produit, soumis au traitement à l'acide, provient du chauffage avec la phosphine à une température ne dépassant pas 80°C, comprise de préférence entre 60° et 80°C, la durée de chauffage, ne dépassant pas 6 heures, étant de préférence de 3 à 5 heures.

8. Procédé suivant une des revendications précédentes, caractérisé en ce que la phosphine employée est une diphosphine chirale.

9. Application du procédé suivant une des revendications 1 à 8 à la préparation d'un catalyseur d'hydrogénation asymétrique.

10. Application suivant la revendication 9, caractérisée en ce que le catalyseur sert à l'hydrogénation d'un composé portant un carbonyle et une fonction amine ou amide.

11. Application suivant la revendication 9 ou 10, caractérisée en ce que l'on hydrogène l'acéto-3 amido-2 butyrate d'alkyle ou d'aryle en vue de la préparation de la thréonine.

**Claims**

1. A method of preparing a chiral catalyst consisting of an organic complex of ruthenium and phosphorus, which comprises heating a complex or ruthenium with a phosphine in an organic liquid which is inert towards the substances present, the heated complex being based on a diene and an allyl compound, wherein the product obtained after this heating is subjected to the action of an acid in an organic solvent.

2. A method according to claim 1 wherein the acid is an inorganic or organic acid, in particular a hydracid or sulfuric, sulfonic, perchloric, phosphoric, fluorosulfonic, acetic, propionic or benzoic acid.

3. A method according to claim 2 wherein the preferred acid is HC1, HBr, HI or $HBF_4$.

4. A method according to any one of claims 1 to 3 wherein the acid in its solvent is at a 0.2 to 3 N solution, and preferably at a 0.5 to 1.5 N solution.

5. A method according to any one of claims 1 to 4 wherein the amount of acid employed is at least 2 equivalents, and preferably from 2 to 4 equivalents, per Ru atom present.

6. A method according to any one of the preceding claims wherein the acid treatment takes place at a temperature of from 0° to 40°C, and in particular of between 15° and 25°C, for from 1/4 hour to 6 hours.

7. A method according to any one of the preceding claims wherein the product subjected to the acid treatment is produced by heating with the phosphine at a temperature not exceeding 80°C, and preferably of between 60° and 80°C, the heating time not exceeding 6 hours and preferably being from 3 to 5 hours.

8. A method according to any one of the preceding claims wherein the phosphine employed is a chiral disphosphine.

9. A use of the method according to any one of claims 1 to 8 for preparing an asymmetric hydrogenation catalyst.

10. A use according to claim 9, wherein the catalyst is used for the hydrogenation of a compound carrying a carbonyl group and an amine or amide function.

11. A use according to claim 9 or claim 10, wherein an alkyl or aryl 3-aceto-2-amidobutyrate is hydrogenated in order to prepare threonine.

**Patentansprüche**

1. Verfahren zur Herstellung eines chiralen Katalysators, der besteht aus einem organischen Komplex aus Ruthenium und Phosphor, das umfaßt Erhitzen eines Ruthenium-Komplexes mit einem Phosphin in einer organischen, gegenüber den vorhandenen Substanzen inerten Flüssigkeit, wobei der erhitzte Komplex sich auf ein Dien und eine Allyl-Verbindung gründet, dadurch gekennzeichnet, daß das infolge des Erhitzens erzeugte Produkt in einem organischen Lösungsmittel der Wirkung einer Säure ausgesetzt wird.

2. Verfahren nach Anspruch 1, dadurch gekennzeichnet, daß die Säure Mineralsäure oder eine organische Säure ist, insbesondere Hydrazid, Schwefelsäure, Sulfonsäure, Perchlorsäure, Phosphorsäure, Fluorsulfonsäure, Essigsäure, Propionsäure oder Benzoesäure.

3. Verfahren nach Anspruch 2, dadurch gekennzeichnet, daß die bevorzugte Säure HCl, HBr, HI oder $HBF_4$ ist.

4. Verfahren nach einem der Ansprüche 1 bis 3, dadurch gekennzeichnet, daß die Lösung der Säure in ihrem Lösungsmittel eine Säure von 0,2 bis 3 N, vorzugsweise von 0,5 bis 1,5 N, ist.

5. Verfahren nach einem der Ansprüche 1 bis 4, dadurch gekennzeichnet, daß die Menge der eingesetzten Säure mindestens zwei Äquivalente auf ein vorhandenes Ru-Atom und vorzugsweise 2 bis 4 Äquivalente beträgt.

6. Verfahren nach einem der vorausgegangenen Ansprüche, dadurch gekennzeichnet, daß die Behandlung mit der Säure bei einer Temperatur von 0 ° bis 40 °C und insbesondere zwischen 15 ° und 25 °C 1/4 Stunde bis 6 Stunden lang stattfindet.

7. Verfahren nach einem der vorausgegangenen Ansprüche, dadurch gekennzeichnet, daß das der Säure-Behandlung unterzogene Produkt entstammt dem Erhitzen mit dem Phosphin bei einer Temperatur, die 80 °C nicht übersteigt, vorzugsweise zwischen 60 und 80 °C, wobei die Dauer des Erhitzens, das 6 Stunden nicht übersteigt, vorzugsweise zwischen 3 und 5 Stunden liegt.

8. Verfahren nach einem der vorausgegangenen Ansprüche, dadurch gekennzeichnet, daß das eingesetzte Phosphin ein chirales Diphosphin ist.

9. Anwendung des Verfahrens nach den Ansprüchen 1 bis 8 zur Herstellung eines asymmetrischen Hydrierungs- Katalysators.

10. Verwendung nach Anspruch 9, dadurch gekennzeichnet, daß der Katalysator der Hydrierung einer Verbindung dient, die ein Carbonyl und eine Amin- oder Amid-Funktion trägt.

11. Verwendung nach Anspruch 9 oder 10, dadurch gekennzeichnet, daß 3-Acetamido-2-alkylbutyrat oder -arylbutyrat hydriert wird, um Threonin herzustellen.